# EUROPEAN PATENT APPLICATION

(11) **EP 4 079 210 A1**
(43) Date of publication of application: **26.10.2022**
(21) Application number: 21169306.4
(22) Date of filing: 20.04.2021
(51) Int. Cl.: A61B 1/00, A61B 1/002, G02B 23/24

(54) **ENDOSCOPE**

(71) Applicant: Weiger, Ulrich, 9462 Montlingen (CH)
(72) Inventor: Weiger, Ulrich, 9462 Montlingen (CH)
(74) Representative: Pfenning, Meinig & Partner mbB

(57) **Abstract**

The invention relates to an endoscope (1, 1'), comprising:
a shaft (2) having a distal end portion (3) and a proximal end portion (4),
a first objective (5) arranged at the distal end portion (3) and having a first direction of view (7), and a first optical axis (21), the first objective (5) being set up to generate an object image in infinity,
a second objective (6) arranged at the distal end portion (3) and having a second direction of view (8), and a second optical axis (22), the second objective (6) being set up to generate an object image in infinity, and
a lens system (10, 30) disposed within the shaft (2) between the distal end portion (3) and the proximal end portion (4), the lens system (10, 30) having an optical axis (9),
wherein the first objective (5) and the second objective (6) are both optically coupled to the same lens system (10, 30) such that first parallel rays from the first objective (5) and second parallel rays from the second objective (6) are input into the lens system (10, 30),
wherein the lens system (10, 30) is
an optical relay system (10) comprising at least one relay group (11,41,61) or a tube lens group (30).

## Description

The present invention relates to an endoscope having a first objective and a second objective projecting to infinity, wherein the first objective and the second objective are both optically coupled to the same lens system such that first parallel rays from the first objective and second parallel rays from the second objective are input into the lens system.

In minimally invasive surgery, rigid endoscopes are commonly used for optical diagnostics as well as for the monitoring of surgical procedures.

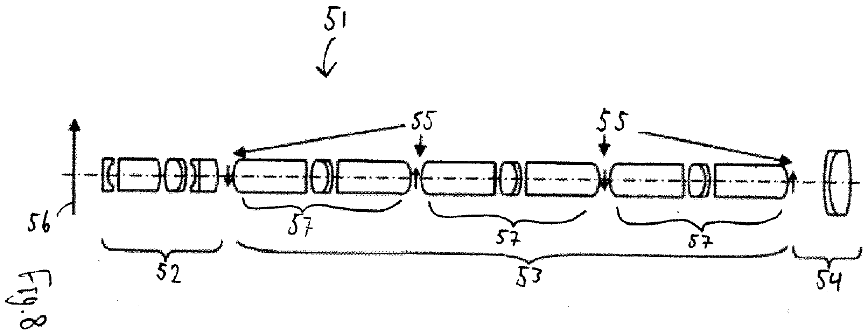

Fig. 8 shows a typical optical layout of a conventional rigid endoscope 51 according to the prior art. The endoscope 51 comprises an objective 52, a relay system 53 and an eyepiece 54. A real intermediate image 55 of the object 56 is formed by the objective 52 and is input into the relay system 53. The relay system 53 transfers the intermediate image 55 to the proximal end portion of the endoscope 1, where the eyepiece 54 allows seeing the image with the naked eye or an attached camera. As shown, the relay system 53 comprises several relay groups 57 each having at least two elongated rod lenses. The relay groups 57 extend the optical pathway of the endoscope 1 and generate several real intermediate images 55 of the object 56. The intermediate images 55 are formed by each relay group 57 outside of the respective relay group 57 and are input into the next relay group 57. Such conventional endoscopic relay systems 53 usually have a telecentric design to avoid vignetting especially when several relay groups 57 are used.

There is an ongoing need to improve endoscopes. For instance, conventional endoscopes can be relatively heavy due to the glass used for the optical components, e.g. the rod lenses described above. For better handling it would be advantageous to reduce the weight of endoscopes. Further, in conventional endoscopes optical components may relatively easy break when the endoscope is bended, e.g. during a medical procedure. On the other hand, such rod lenses can be manufactured in a relatively straightforward manner, and have been successfully used in many conventional endoscopes.

To adapt the endoscope to various applications and associated practical needs, it would be also advantageous to increase freedom of design of the endoscope, in particular of its optical components.

There are endoscopes with different direction of view available, which allows the clinician to investigate different directions of view of a body passage. However, in some instances, the direction of view needs to be changed during an operation forcing the clinician to exchange the endoscope, the associated light cable and possibly also the camera. To avoid this, efforts have been made worldwide to develop rigid endoscopes with a variable direction of view.

For instance, a solution for an endoscope having a switchable direction of view is the so-called swing prism endoscope. The optical layout of swing prism endoscopes differs from the standard layout by a swing prism at the tip of the endoscope (for changing the direction of view) and an erection prism at the proximal end the endoscope. However, the swing prism endoscope has some disadvantages, such as a relatively small field of view, a relatively low image quality, and a comparatively low light throughput. Furthermore, usually there is no zero degree direction of view realizable in swing prism endoscopes.

In order to solve the problems associated with swing prism endoscopes, a periscopic endoscope was developed by Ulrich Weiger in his PhD thesis "Entwicklung eines Endoskops mit variabler Blickrichtung für die Laparoskopie", TU Berlin, 2013. While said periscopic endoscope overcomes various disadvantages of the swing prism endoscope, there are disadvantages associated with the periscopic endoscope as well, such as a complex correction of image rotation and a relatively complex mechanical system rendering it difficult to scale to smaller endoscope diameters.

Some exemplary endoscopes are known from WO 97/27798 A1, US 2002/082476 A1, JP H05 341207 A and US 6 361491 B1.

It is an object of the present invention to solve at least one of the foregoing issues or problems.

This object is solved by an endoscope as defined in claim 1. Further aspects, developments and embodiments are described in the dependent claims and the following description.

According to the present disclosure, an endoscope is provided, comprising inter alia:
a shaft having a distal end portion and a proximal end portion,
a first objective arranged at the distal end portion (3) and having a first direction of view, and a first optical axis, the first objective being set up to generate an object image in infinity,
a second objective arranged at the distal end portion and having a second direction of view, and a second optical axis, the second objective being set up to generate an object image in infinity, and
a lens system disposed within the shaft between the distal end portion and the proximal end portion, the lens system having an optical axis,
wherein the first objective and the second objective are both optically coupled to the same lens system such that first parallel rays from the first objective and second parallel rays from the second objective are input into the lens system,
wherein the lens system is
an optical relay system comprising at least one relay group or
a tube lens group.

In the context of the present disclosure, the expression "parallel rays" may mean that the marginal rays are parallel to the corresponding chief rays. More specifically, the paraxial marginal ray angle relative to the chief ray may be 0°.

The space between the objectives and the lens system may be referred to as infinity space, wherein the marginal rays are parallel to the corresponding chief rays. In said space there are no intermediate real images of the object generated, instead the images are formed by the objectives at a plane in infinity. In other words, the image distance of the objectives is set to infinity. Thus, both objectives form images at a plane in infinity, which means all rays coming from an object point are at least approximately parallel to each other at the image side of the objective, i.e. in the infinity space, therefore the marginal rays are parallel to the corresponding chief rays. In practice, due to aberrations or other optical errors the rays may not be perfectly parallel to each other. A deviation of at most 5° or at most 3° or at most 1° or less than 1° may still fall within the meaning of the expression "parallel".

The parallel rays generated by the objectives coming from respective object points are input into the lens system. In the following, the rays input into the first and second objectives and output from the first and second objectives may be referred to as first rays and second rays, respectively. Rays coming from one specific object point, input into the first or second objectives, and output from the first or second objectives may be defined as first parallel rays and second parallel rays, respectively.

The infinity space between the objectives and the lens system allows a decentering of the respective objective axis with respect to the optical axis of the lens system. Thus, both objectives share one lens system, to which their optical axes may be decentered. For instance, the first optical axis and the second optical axis can be offset from the optical axis of the lens system.

Furthermore, the distance between the respective objective and the lens system does not have to obey a predetermined distance, as the object images are formed at a plane in infinity. Thus, the infinity space allows a variable distance between the objectives and the lens system. In some embodiments, a distance between the first objective and the lens system differs from a distance between the second objective and the lens system. The infinity space between the both objectives and the lens system also allows additional optical components to be inserted between the objectives and the lens system as long as they do not substantially change the parallelism of the first and second parallel rays. Such optical components may comprise optical filters, polarisers, illuminators, beamsplitters and/or shutters. It is noted that the space between the objectives and the lens system (i.e. relay system or tube lens) can be void of any focussing elements such as lenses that could change the parallelism of the first and second parallel rays. In some embodiments, the first and second rays coming from the first and second objectives are directly input into the lens system.

Typically, the endoscope has a first fixed focus corresponding to a first focal plane of the first objective and/or a second fixed focus corresponding to a second focal plane of the second objective. Thus, only objects disposed in the first focal plane or the second focal plane may be result in sharp, well-focussed and clear images that can be viewed using a connected eyepiece or a camera. If a user such as a clinician or physician wants to change position of the focus plane, the endoscope has to be moved e.g. by axially pushing or retracting the endoscope. According to an implementation, the camera may have a focussing lens for modifying the focus plane.

The first and/or second objectives may comprise a plurality of lenses. For instance, each objective may comprise an input lens group and an output lens group. Lens group may refer in the context of the present disclosure to at least one lens or two lenses or more that are preferably in firm contact with each other. The input lens group may face the object, while the output lens group usually faces the lens system (i.e. the relay system or the tube lens). In some embodiments, the input lens group of the first and/or second objectives may have a negative focal length. The output lens group of the first and/or second objectives may have a positive focal length. At least one of the first and/second objectives may comprise at least one mirror and/or at least one prism arranged between the input lens group and the output lens group. By providing a prism and/or mirror in the objective, the direction of view of said objective may be changed.

Generally, by changing the arrangement of the first objective and/or the second objective, in particular of the optical components relative to each other, the first direction of view, the second direction of view, the first optical axis and the second optical axis may be altered. Thus, the optical configuration of the first objective and/or the second objective may be such that certain specifications are met. In the following, examples for such specifications are described.

For instance, the first direction of view and the second direction of view are not parallel to each other. In this case, the two objectives at the distal end portion enable two different directions of view. To inspect a body passage a medical caregiver may switch between the two view directions using only one endoscope and without having to exchange the endoscope during the medical procedure.

In alternative embodiments, the first direction of view and the second direction of view are parallel to each other. This may be particularly useful for stereoscopic endoscopes.

In some instances, the first optical axis is defined by a lens of the first objective (e.g. the output lens group) most proximate to the lens system and the second optical axis is defined by a lens of the second objective (e.g. the output lens group) most proximate to the lens system.

For instance, the first direction of view, the second direction of view and the optical axis of the lens system may be tilted relative to one other. The first direction of view, the second direction of view and the optical axis of the lens system may not lie in the same plane. The optical axis of the lens system may not be parallel to the first direction of view and/or the second direction of view. Optionally, the first optical axis may be parallel with the first direction of view and/or the second optical axis may not be parallel with the second direction of view. The optical axes of the two objectives may define a plane, while the axis of the direction of view of the first and/or the second objective does not coincides with this plane.

Optionally, the first direction of view forms a plane with the first optical axis of the first objective, which is parallel and not coincident with a plane formed by the second direction of view and the second optical axis of the second objective. The first optical axis and the second optical axis may each form a plane with the optical axis of the lens system, wherein these planes are not perpendicular to the planes mentioned above.

In some examples, the first and second objectives are fixed relatively to one other. Furthermore, the first and second objectives may be fixed relatively to the shaft. For example, all optical components of the first and second objectives are fixed relatively to the shaft. For instance, the first objective, the second objectives and/or the lens system do not comprise any moving or rotating parts. By providing fixed objectives without moving or rotating parts, the endoscope can be designed in a particularly robust manner. Thus, the two different directions of view may be fixed relative to the shaft of the endoscope.

It should be mentioned that the endoscope may comprise more than two objectives as well.

In the following, the endoscope having the relay system is described.

The relay system typically extends the optical pathway. Sometimes the relay system also inverts the image. The relay system may preferably be an afocal system. Generally, an afocal system (a system without focus) may be regarded as an optical system that produces no net convergence or divergence of a beam, i.e. has an infinite effective focal length.

The relay system may include one or more conventional lenses and/or achromatic doublets. In some instances, the relay system comprises at least one relay group or a plurality of relay groups. The number of relay groups may be selected depending on specifications such as the length and diameter of the inserted section of the endoscope and the brightness and the like of the optical system. The plurality of relay groups may be arranged in series such that light exiting a relay group may enter the next relay group. For instance, if at least two relay groups are envisaged, the endoscope may configured such that the first and second parallel rays are coupled out of the first relay group and are input into the next relay group. Each relay group may be configured to invert or rotate the image and to extend the optical pathway. In preferred embodiments, each relay group has the same optical layout. However, in some instances, various relay groups having different optical layouts may be used.

Optionally, at least one relay group is axial symmetric around a symmetry axis, which is perpendicular to the optical axis of the relay group or relay system. The relay group may be mirror-symmetrical with respect to a mirror plane, which is perpendicular to the optical axis of the relay group and/or the relay system. Often, relay groups having said symmetry axis and/or symmetry plane can be manufactured more cheaply than relay groups having an asymmetric design. Optionally, the symmetry axis and/or symmetry plane may be disposed in the center of the relay group. More specifically, the symmetry axis may be disposed in the center of the optical cavity of the relay group. Typically, the symmetry axis and/or symmetry plane does not coincide with a lens or lens group. Sometimes, an intermediate image formed at an image plane within the relay group substantially coincides with the symmetry axis and/or symmetry plane. In some embodiments, the symmetry axis and/or symmetry plane coincide with a symmetric lens or lens group. In these embodiments, the intermediate image can be formed within the lens or lens group.

It may be envisaged that the relay system has a total magnification of -1 or 1. That is, the relay system does not alter the width or the height of the beam. In some instances, the magnification of the at least one relay group is -1 or 1. For instance, each relay group of the relay system has a magnification of -1.

Optionally, the relay system is configured to generate first and second intermediate images from the first and second rays, respectively. Usually, the first and second intermediate images are generated within (i.e. inside) the relay system and at least partially overlay. For instance, the endoscope is configured such that the first and second intermediate images concentrically overlay within the relay system. The images of both objectives may be concentrically overlaid in the intermediate images of the relay system, while the pupils of both objectives are decentered in the pupil plane of the relay system.

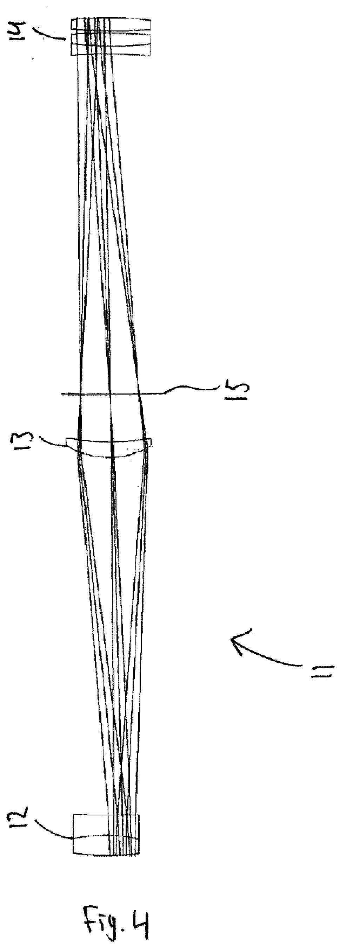

The endoscope may be configured such that the first parallel rays and the second rays are coupled out of the relay system at the proximal end portion. In particular, the first rays and the second rays may be spatially separated from each other at the proximal end portion (pupil plane). Further, at the proximal end portion the first parallel rays and the second parallel rays coming from the first and second objectives and coupled out of the relay system still have marginal rays, which are parallel to their corresponding chief rays. In particular, the first parallel rays and the second parallel rays may be spatially separated from each other at the proximal end portion. To form an image in front of an eyepiece or for a proximally located image sensor an additional lens group may be used. Said lens group may comprise, for example, the input lens group 12 and the field lens 13 as shown in Fig. 4. Alternatively, an additional tube lens forms the intermediate image at the proximal end. Alternatively the relay system is designed and corrected in such way, that the angle of the parallel rays coupled out of the relay system is suitable to directly see the image with naked eye or an attached camera.

The shaft of the endoscope may be rigid. Usually, rigid endoscopes give higher quality images, are easier to use and are less expensive than flexible endoscopes of similar quality. However, in alternative embodiments, the shaft of the endoscope may be flexible, e.g. may be configured to flex or articulate around an axis. The shaft may also have at least one rigid portion and at least one flexible portion. For instance, a relay group having relatively rigid rod lenses may be arranged at the rigid portion of the shaft. Additionally or alternatively, a relay group having a cavity spanning at least 40% of the total length of the relay group may be disposed at the flexible portion of the shaft.

In conventional rigid endoscopes it is common to use rod lenses in the relay system to reduce air gaps and increase the possible numeral aperture in the system for the same amount of relays. To get the image of the objective through the relay system without significant vignetting, the optical design of such conventional endoscopes is usually telecentric within the relay system. This means that the chief rays coming from the object are parallel to the optical axis. The disadvantage of such conventional setups is that the position of the "stop" surface is restricted to create a telecentric design (e.g the exit pupil must be at infinity to obtain a telecentric image space). As the position of the stop influences several aberrations, its position can be only partly used to reduce aberration when its position is already defined by telecentricity. It is noted that, in conventional endoscopes, intermediate images are generated outside the respective relay groups, i.e. after the output lens group of the respective relay group and before the input lens group of the next relay group.

According to an aspect of the present disclosure, the chief rays coming from different object points and output from the objectives are usually not parallel to each other and/or are typically not parallel to the optical axis of the lens system. The only exception may be the chief ray coming from the middle of the object. Said chief ray may be parallel to the optical axis of the lens system.

Moreover, according to some embodiments the relay system and/or at least one relay group can comprise an input lens group, an output lens group and a cavity defined between the input lens group and the output lens group. The cavity may have a length spanning at least 30%, 40%, 50%, 60%, 70% or 80% of a total length of the relay group. Optionally, exactly one or at least one field lens is disposed in the cavity. The relay group can have an asymmetric or symmetric design. The field lens may be a positive-powered lens or group of lenses. The field lens may be used to direct the chief rays into the next lens (e.g. the output lens group) of the respective relay group and therefore may reduce the vignetting without telecentricity. Thus, by using the relay system with the field lens as described above, the stop position may be more variable and can be used for a better aberration control. In this way, the use of rod lenses may be obviated. Thus, the endoscope, the at least one relay group and/or the relay system may lack any rod lenses.

Thus, by providing the cavity and preferably the field lens, the weight of the endoscope can be reduced as compared to systems having rod lenses. Furthermore, the risk of breaking optical components such as rod lenses may be reduced when the endoscope is subject to mechanical stress such as bending.

According to some embodiments, the at least one relay group consists of a single input lens and a single output lens. In other words, both lenses are singlet lenses and relay group does not include any further lenses or other optical elements. A cavity void of optical elements may extend between the input lens and the output lens, i.e. extend from the input lens to the output lens. The cavity may have a length spanning at most 50%, 40%, 30%, 20% or 10% or less of a total length of the relay group. Optionally, the input lens is a rod lens and/or the output lens is a rod lens. The input lens and/or the output lens may be biconvex or plano-convex. The input lens and the output lens can be made of the same material. The relay group can have a symmetric design. For instance, the relay group may have the above mentioned mirror symmetry. Optionally, the input lens and the output lens are identical lenses, which are turned 180° relatively to one other. When the relay group includes only two lenses, namely the input lens and the output lens, the optical design can be manufactured in a simple manner, which may help reduce complexity of the system. In some embodiments, the at least one relay group is configured such that first and second intermediate images are generated within the at least one relay group, in particular between the input lens and the output lens thereof.

In some implementations, the relay system may comprise at least two relay groups each having a different optical layout. For instance, one of the relay groups may comprise the input lens group, the output lens group, and the above mentioned field lens disposed between the input lens group and the output lens group, while another relay group consists only of the above mentioned input lens and output lens, and the cavity between the input lens and the output lens is void of optical elements. In alternative embodiments, each relay group of the relay system has the same optical layout.

The relay system typically has an entrance side at which the first and second rays enter the relay system. Further, the relay system typically has an exit side at which the first and second rays exit the relay system. As indicated above, the optical design of the relay system may be such that the entrance side and/or the exit side are not telecentric. This means that the chief rays are not parallel to the optical axis in front of or behind the relay system. Further, chief rays coming from different object points may be not parallel to each other. In this way, the optical design freedom can be enhanced, which in turn may have the effect that the optical design can be improved with regard to telecentric systems.

Optionally, the first rays and/or the second rays input in the relay system are converted within the relay system to first intermediate images and/or second intermediate images, respectively. For instance, each relay group may be configured to generate first and second intermediate images within the respective relay group. The first and/or second intermediate images may be generated within the respective relay group after the field lens. In particular the first and/or second intermediate images may be generated such that the first and/or second intermediate images have a size, which is smaller than a maximum diameter of all of the relay group's lenses. Furthermore, all intermediate images generated in the relay system may have the same paraxial size.

In various embodiments, the endoscope may further comprise a tube lens and/or an eyepiece. The eyepiece may be disposed at the proximal end portion of the endoscope. The number of relay groups may be selected such that the final image projected by the tube lens and/or eyepiece is in an upright position. Usually this means an uneven number of first and/or second intermediate images generated in the relay system. For instance, the total number of relay groups may be uneven. An optical sensor such as a camera may be connected to the eyepiece. The optical sensor may also be used instead of the eyepiece. In this case, a tube lens may be disposed between the relay system and the optical sensor to project the final images onto the optical sensor. As mentioned above, to form an image in front of the eyepiece or for a proximally located image sensor an additional lens group may be used. Said lens group may comprise, for example, the input lens group 12 and the field lens 13 as shown in Fig. 4. Alternatively, an additional tube lens forms the intermediate image at the proximal end. Alternatively the relay system is designed and corrected in such way, that the angle of the parallel rays coupled out of the relay system is suitable to directly see the image with naked eye or an attached camera. In this case, an additional relay group may be envisaged to get an upright image, or the camera may be configured to flip the image by 180°.

The endoscope may further comprise a shutter having at least two operational states being configured to selectively block the first or second rays. The shutter can comprise e.g. a rotating disc, a shifting mask or other suitable means for selectively blocking the first and second rays. The shutter may be arranged, for example, within the infinity space or after the relay system. In particular, the shutter may be arranged in the last pupil plane at the proximal end of the relay system. By partially blocking the pupil plane of the relay system a user can switch between the images of the two objectives. Alternatively, the shutter may be disposed between two relay groups of the relay system. The shutter can also be arranged between the relay system and the tube lens. It can also be arranged between the objective and the relay system or the tube lens.

Instead of the relay system, the infinite images or first and second rays produced by the objectives, wherein the marginal rays may be parallel to the corresponding chief rays, are input into a tube lens. In the following, the endoscope comprising the tube lens is described.

In some embodiments, the endoscope comprises an image sensor arranged at the distal end portion of the shaft. The tube lens group may be arranged between the first and second objectives and the image sensor. The tube lens group may be configured to project a first final image and/or a second final image onto the image sensor. Thus, the image sensor may be disposed in the focal plane of the tube lens group.

The tube lens group may comprise solely one lens or a plurality of lenses, which may be in firm contact with each other.

In many rigid endoscopes with integrated image sensor, the image sensor is placed on the image side of the objective instead of using a relay system. With the two decentered objectives as described above, one tube lens group is disposed in front of the image sensor. In this way, a larger image sensor can be used, which has advantages in terms of e.g. signal to noise ratio or availability.

The shutter may be disposed between the objectives and the tube lens group. If the first and second objectives have the same direction of view, the shutter may switch between left and right creating a 3D image (stereoscopic image) using just one image sensor. By partially blocking the pupil plane of the objectives a user can switch between the images of the two objectives.

Features that have been described with regard to the endoscope with the relay system but are not related to the relay system thereof may be combined with the endoscope having the tube lens group as long as they do not contradict each other. Furthermore, features that have been described with regard to the endoscope having the tube lens group but are not related to the tube lens group thereof be combined with the endoscope having the tube lens group as long as they do not contradict each other.

Various objects and advantages of the present invention will become apparent to those skilled in the art from the following detailed description of embodiments, when read in light of the accompanying drawings.
- Fig. 1: shows schematic representations of two endoscopes.
- Fig. 2: schematically illustrates several optical components of an endoscope.
- Fig. 3: schematically illustrates several optical components disposed in a front/distal tip of the endoscope of Fig. 2.
- Fig. 4: schematically illustrates a relay group of one of the endoscopes depicted in Figs. 1-3.
- Fig.5: schematically illustrates a part of the endoscope depicted in Figs. 1-3.
- Fig.6: schematically illustrates a detail of the endoscope shown in Figs. 1-3 and further including a shutter.
- Fig. 7: schematically illustrates a front/distal tip of an endoscope.
- Fig.8: schematically illustrates a conventional endoscope.
- Fig.9: schematically illustrates a further example of a relay group.
- Fig. 10: schematically illustrates a part of a further endoscope.
- Fig. 11: schematically illustrates a further example of a relay group used in the endoscope shown in Fig. 10.

In the following, recurring and similar features in this and in the subsequent representations are provided with the same reference numerals.

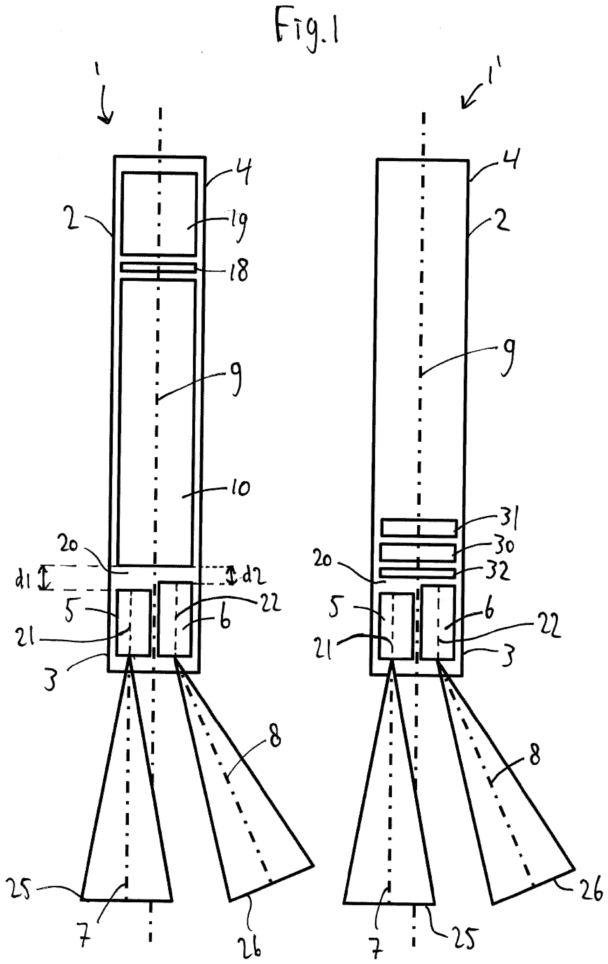

In Fig. 1 two endoscopes 1, 1' are schematically illustrated. The endoscopes 1, 1' comprise an elongated shaft 2 having a distal end portion 3, a proximal end portion 4 and an intermediate portion extending therebetween. The endoscopes 1, 1' further comprise a first objective 5 and a second objective 6 which are disposed in the distal end portion 3 of the shaft 2. The shaft 2 may be rigid along its entire length. In alternative embodiments, the shaft 2 may have at least one rigid portion and at least one flexible portion. In its flexible portion(s), the shaft 2 may be configured to flex or articulate around a longitudinal axis.

The first objective 5 has a first direction of view 7, a first focal plane 25 on the object side coincident with the object plane, and a first optical axis 21. The second objective 6 has a second direction of view 8, a second focal plane 26 on the object side coincident with the object plane, and a second optical axis 22. The first and second objectives 5, 6 are set up to generate an object image in infinity. The objectives 5, 6 are typically disposed inside the shaft 2. The field of view of each objective 5, 6 is indicated in Fig. 1 by a cone accompanying the respective direction of view 7, 8.

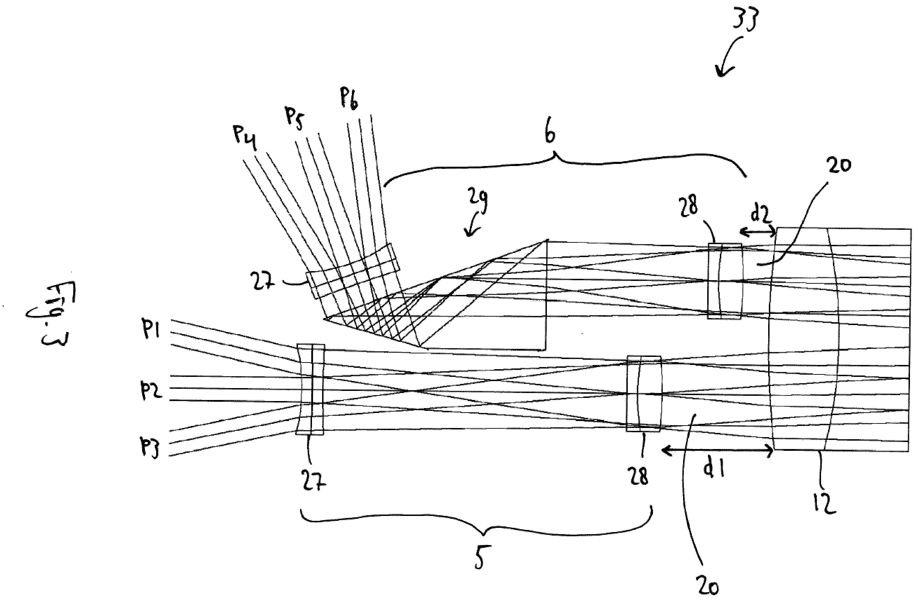

Reference is additionally made to Fig. 3, which shows several optical components disposed in distal end portion 3 of the endoscope, which is sometimes called the front tip 33 of the endoscope 1. It should be mentioned that the optical layout of the objectives 5, 6 shown in Fig. 3 can also be used for the objectives 5, 6 of endoscope 1'.

The first and second objectives 5, 6 include a plurality of lenses. In the embodiment depicted, each of the objectives 5, 6 includes an input lens group 27 and an output lens group 28. The input lens groups 27 of the objectives 5, 6 can be of the same design or may each have a different optical design. The output lens groups 28 of the objectives 5, 6 may have the same optical design or differ from each other. The input lens group 27 gathers light coming from the object being observed, while the output lens group 28 faces the lens system 10, 30. In some examples, the input lens group 27 of the objectives 5, 6 has a negative focal length, while the output lens group 28 of the objectives 5, 6 has a positive focal length. The second objective 6 comprises a prism assembly 29 arranged between the input lens group 27 and the output lens group 28. The prism assembly 29 can comprise an arrangement of reflective and/or refractive prisms. By virtue of the first lens group 27 and the prism assembly 29 the second direction of view 8 of the second objective 6 differs from the first direction of view 7 of the first objective 5. In particular, the first direction of view 7 and the second direction of view 8 are not parallel to each other.

The first optical axis 21 of the first objective 5 is defined by the optical axis of the output lens group 28 thereof. Further, the second optical axis 22 of the second objective 6 is defined by the optical axis of the output lens group 28 thereof. The first and second optical axes 21, 22 may be parallel to each other, but do not overlap each other. Furthermore, the first and second optical axes 21, 22 are parallel to the optical axis of the lens system 10, 30 and the longitudinal axis of the shaft.

In the depicted embodiment, the optical axis 21 and the first direction of view 7 are parallel to each other. Furthermore, an axial extension the first optical axis 21 coincides with an axial extension of the direction of view 7. According to alternative embodiments, the first direction of view 7 is not parallel to the first optical axis 21. In contrast, the optical axis 22 is not parallel to the second direction of view 8. As follows from the above, the optical axes 21, 22, the directions of view 7,8 can be varied and adapted to practical needs.

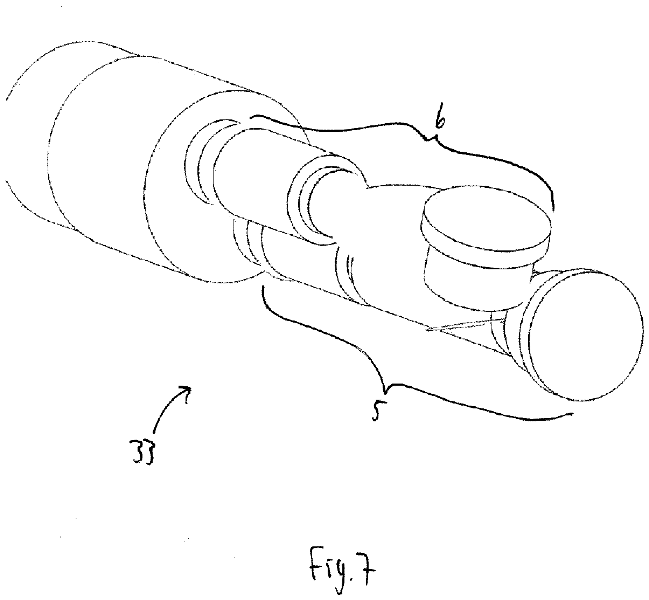

As can be seen from Fig. 7, which shows a front tip 33 of exemplary endoscopes 1, 1', the second direction of view 8 is tilted with respect to the optical axis 9 of the lens system 10, 30 and the longitudinal axis of the shaft 2. Consequently, the first direction of view 7, the second direction of view 8 and the optical axis 9 do not lie in the same plane. In a further example, the first direction of view 7 and the second direction of view 8 are tilted with respect to the optical axis 9 of the lens system 10, 30 and the longitudinal axis of the shaft 2.

In alternative embodiments (not shown), the first direction of view and the second direction of view are parallel to each other. This may be particularly useful for stereoscopic endoscopes.

Optionally, the first direction of view 7 forms a plane with the first optical axis 21 of the first objective 5, which is parallel and not coincident with a plane formed by the second direction of view 8 and the second optical axis 22 of the second objective 6. The first optical axis 21 and the second optical axis 22 may each form a plane with the optical axis 9 of the lens system 10, 30 (i.e. relay system 10 or tube group 30), wherein these planes are usually not perpendicular to the planes mentioned above.

The optical components of the first and second objectives 5, 6 are fixed relatively to one other and to the shaft 2. Thus, the objectives 5, 6 do not include any moving or rotating parts.

Even though the endoscopes 1, 1' shown comprise two objectives 5, 6, it should be mentioned that the endoscopes 1, 1' may also comprise more than two objectives.

The endoscopes 1, 1' further include a lens system 10, 30 disposed within the shaft 2 between the distal end portion 3 and the proximal end portion 4, the lens system 10, 30 having an optical axis 9. The optical axis 9 is parallel to the longitudinal axis of the shaft 2 and optionally coincides with the longitudinal axis of the shaft 2. Both objectives 5, 6 share the same lens system 10, 30 and are optically coupled thereto. The optical axes 21, 22 are parallel to the optical axis 9 and decentered with respect to the optical axis 9.

Both objectives 5,6 form images at a plane in infinity, therefore the object to be observed is at a plane coincident with the object side focus plane 25, 26, which means for all rays p1, p2, p3, p4, p5, p6 coming from a specific point in the object plane (focus plane 25, 26) the marginal rays are parallel to the corresponding chief ray. Light rays coming from the objectives 5, 6 are directly input in the lens system 10, 30. In particular, since the focal planes 25, 26 are coincident with the corresponding object planes, the objectives 5, 6 generate first and second rays, where the marginal rays are parallel to the chief ray, respectively, that are directly input into the lens system 10, 30.

The space between the objectives 5, 6 and the lens system 10, 30 defines an infinity space 20, as the image distance of both objectives 5, 6 is set to infinity. The term infinity space 20 refers to the production of a flux of light rays, where the marginal rays are parallel to their corresponding chief rays after passing through the respective objectives 5, 6. For instance, in Fig. 3, groups of light rays p1, p2, p3, p4, p5, p6 coming from respective object points are indicated. For each group of rays p1, p2, p3, p4, p5, p6 coming from a respective object point the marginal rays are parallel to the corresponding chief ray at the image side of the objectives 5, 6, i.e. in the infinity space 20.

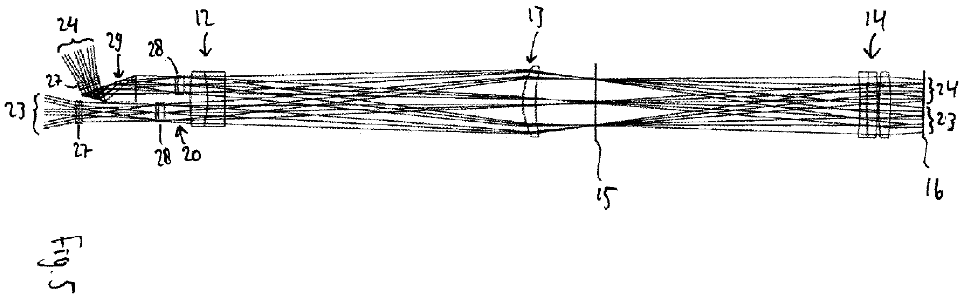

The infinity space 20 allows a variable distance between the objectives 5, 6 and the lens system 10, 30. This may improve freedom of design of the optical components of the endoscopes 1, 1', especially of the objectives 5, 6. This can be very helpful since the space in the distal end portion 3 of the shaft is typically constricted. For example, according to Figs. 1, 2, 3, 5 and 7, a distance d1 between the first objective 5 and the lens system 10, 30 differs from a distance d2 between the second objective 6 and the lens system 10, 30. As the lenses usually have an exit surface with an arced shape, the distances d1, d2 may refer to the smallest distance between the objectives 5, 6 and the lens system 10, 30, respectively.

As can be seen from Fig. 3, chief rays coming from different object points and output from the objectives 5, 6 are parallel neither to each other nor to the optical axis 9 of the lens system 10, 30.

In accordance with the endoscope tip 33 shown in Fig. 7, to use the available space of the two objectives 5, 6 in the endoscope tip 33 more efficiently, the two objectives 5, 6 can be arranged such that the optical axes 21, 22 of the two objectives 5, 6 define a plane, wherein at least one direction of view 7, 8 does not lie in this plane. Further, the optical axes 21, 22 of the two objectives 5, 6 and the optical axis 9 of the lens system 10, 30 do not need to be in the same plane.

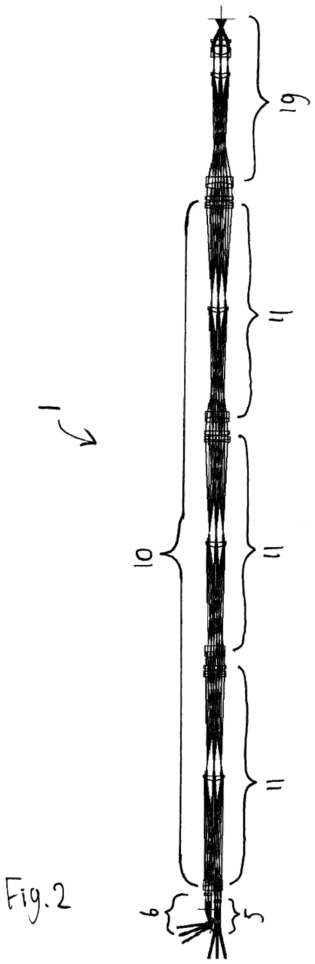

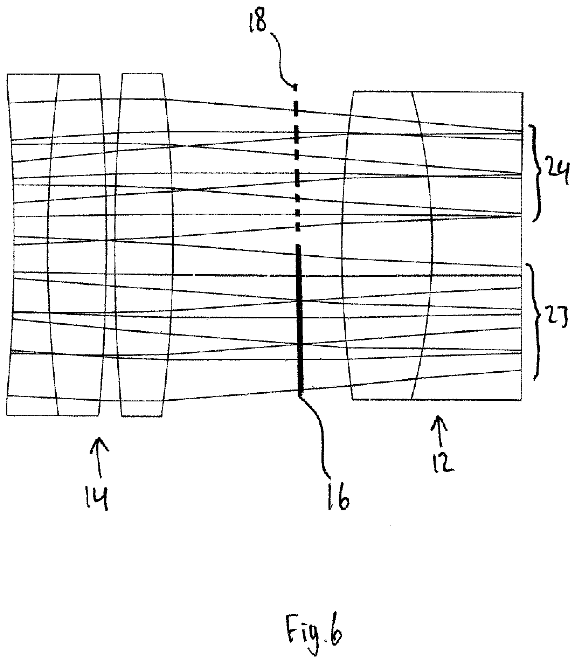

In the following, reference is made to the endoscope 1 of Fig. 1, parts of which are shown in Figs. 2-7.

The endoscope 1 comprises a relay system 10 in order to extend the optical pathway of the endoscope 1 within the shaft 2. The first objective 5 and the second objective 6 are optically coupled to the relay system 10 such that the rays output from the objectives 5,6 are directly input into the relay system 10, i.e. there are no optical components disposed between the objectives 5, 6 and the relay system 10.

The relay system 10 is afocal and has a magnification of -1. The relay system 10 includes a plurality of relay groups 11 that are arranged in series. Each relay group 11 has the same optical layout. For instance, each relay group 11 has a magnification of -1. The total number of relay groups 11 is uneven. Each of the relay groups 11 may comprise an input lens group 12, a field lens 13 and an output lens group 14. The field lens 13 may be a positive-powered lens (e.g. singlet) or group of lenses (e.g. doublet). Typically the input lens group 12 and/or the output lens group 14 are doublets each having a positive focal length and being arranged close to a pupil plane. The lenses of the relay system 10 are selected such that the first and second intermediate images are generated from the first and second rays coming out of the first objective 5 and the second objective 6, respectively, wherein the intermediate images are generated at an intermediate image plane 15 inside the relay groups 11.

The field lens 13 is placed within each relay group 12 of the relay system 10 close to the intermediate image 15 to solve the vignetting issue without telecentric design. This field lens 13 projects the pupil from the front part into the rear part of the relay group 11. The design of this relay system 10 furthermore allows a lens design without rod lenses in contrast to conventional endoscopic relay system (see Fig. 8), where rod lenses are uses to get larger apertures through the relay system. The advantage of this relay design having a field lens 13 and without rod lenses is a reduced weight and a reduced risk of broken lenses.

Inside the relay system 10, the chief rays coming from different object points are not parallel to each other and also not parallel to the optical axis 9 of the relay system 10.

The images of both objectives 5, 6 are concentrically overlaid in the intermediate image plane 15 of each relay group 11 of the relay system 10, while the pupils of both objectives 5, 6 are spaced from each other and decentered in the pupil plane 16 of the relay system 10. The size of the intermediate images at the intermediate image plane 15 and the size of the pupil plane 16 are smaller than the overall diameter of the lenses of the relay system 1. By blocking the pupil plane 16 in front of an input lens group 12 or an output lens group 14 of any relay group 11 of the relay system 10 partly e.g. with a mask on a rotatable disk, a shifting mask or a shutter 18, one can switch between the images of the two objectives 5 and 6. Alternatively, the shutter 18 may be also disposed between the two objectives, 5,6 and the relay system 10 (not shown).

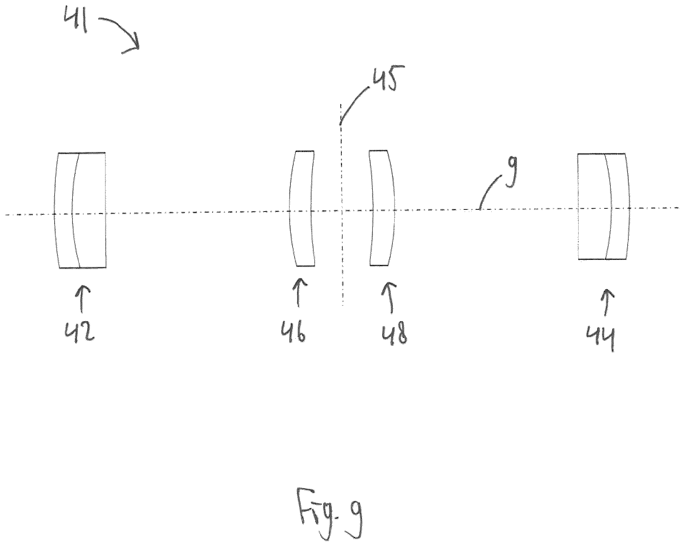

Fig. 9 shows an alternative embodiment of a relay group 41 having a symmetric design, which can be used instead of relay group 11 or in addition to relay group 11. Thus, in some embodiments the relay system 10 may comprise a plurality of relay groups 41 that are arranged in series. Optionally, the relay system 10 may comprise at least one relay group 11 and at least one relay group 41 that are arranged in series. When more than one relay group 41 is envisaged in the relay system 10, each relay group 41 of the relay system 10 may have the same symmetric optical layout. The total number of relay groups 41 comprised in the relay system 10 may be uneven. Similar to relay group 11, the relay group 41 may comprise an input lens group 42 and an output lens group 44. Preferably, the relay group 41 has a magnification of -1 or 1 and is afocal. Between the input lens group 42 and the output lens group two lenses 46, 48 may be disposed, which may be field lenses. The lenses 46, 48 may be identical with respect to their optical properties, however, the lens 48 is rotated by 180 degrees with respect to the optical axis 9. The relay group 41 is axial symmetric around a symmetry axis 45 or symmetry plane, which is perpendicular to the optical axis 9 of the relay system 10. As shown in Fig. 9, the symmetry axis 45 or similarly, the symmetry plane, may be disposed between lenses 46, 48, preferably at the center of the relay group 41. In the embodiment shown, the symmetry axis 45 does not coincide with a lens or lens group. In other embodiments, the symmetry axis or symmetry plane coincides with a lens or lens group, which have a symmetric design. In this case, the material of the lens or lens group should be selected to have a high degree of purity.

In the embodiment of Fig. 9, only two lenses 46, 48 are arranged between the input lens group 42 and the output lens group 44. In some embodiments, there may be even more lenses disposed in the cavity of the relay group 41 as long as the relay group 41 has a magnification of -1, and is both afocal and axial symmetric around the symmetry axis 45. The symmetric design of the relay group 41 allows to reduce the amount of different lens types within the endoscope, which may result in less complexity in the production and reduced costs. Typically, the lenses 46, 48 are singlets, have a positive focal length, a relatively low refraction index and are close to an intermediate image. Typically the lenses groups 42, 44 are doublets each with a positive focal length and being arranged close to a pupil plane. Preferably, the lens groups and lenses 42, 44, 46, 48 of the relay group 41 are selected and arranged such that the first and second intermediate images are generated from the first and second rays 23, 24 at an intermediate image plane inside the relay group 41, wherein the intermediate image plane substantially coincides with the symmetry axis 45.

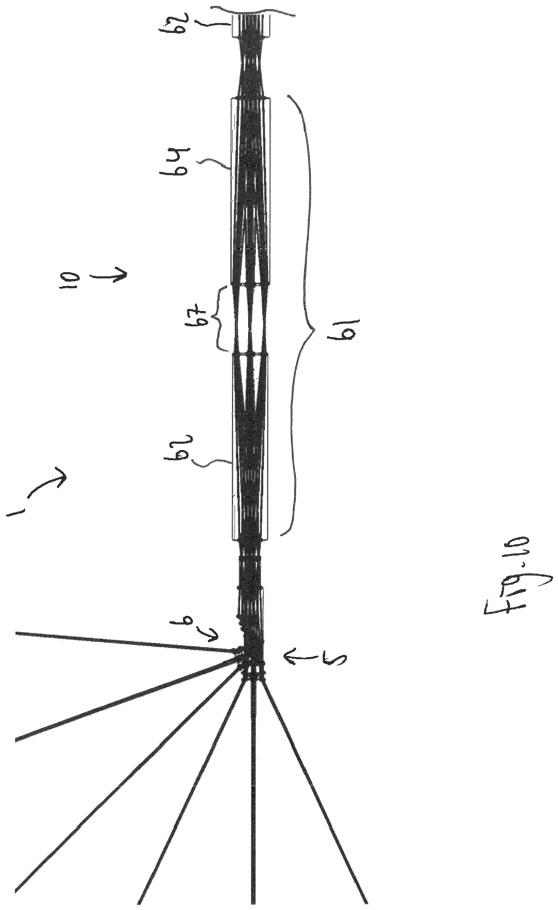

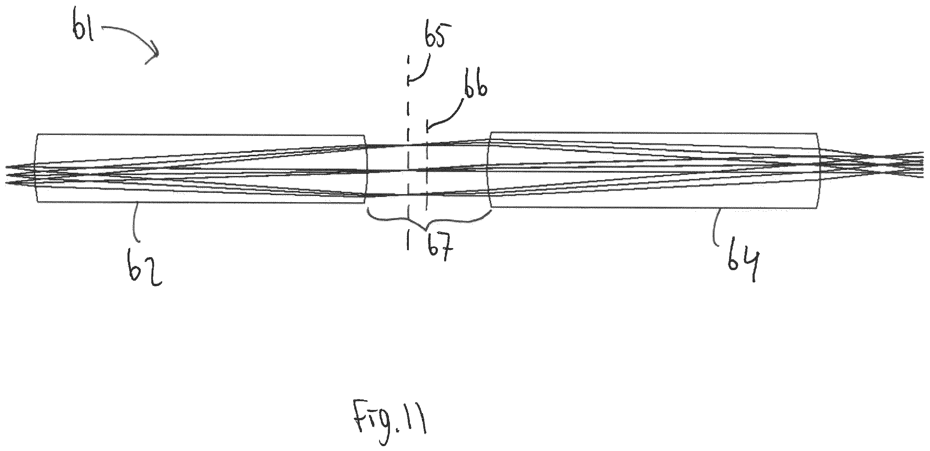

Fig. 10 shows a part of an endoscope 1 having at least one relay group 61. Fig. 11 shows said relay group 61. The relay group 61 has a symmetric design, which can be used in the relay system 10 of endoscope 1 instead of relay groups 11, 41 or in addition to relay group 11 and/or relay group 41. Thus, in some embodiments the relay system 10 may comprise a plurality of relay groups 61 that are arranged in series. Optionally, the relay system 10 may comprise at least one relay group 61 and at least one relay group 11, 41 that are arranged in series. When more than one relay group 61 is envisaged in the relay system 10, each relay group 61 of the relay system 10 may have the same symmetric optical layout. The total number of relay groups 61 comprised in the relay system 10 may be uneven. The relay group 61 consists of one single input lens 62 and one single output lens 64, which are both configured as singlets. The input lens 62 and the output lens 64 may be both envisaged as monolithic elongated rod lenses (singlet rod lenses), preferably made of the same material. In some instances, the input lens 62 and/or the output lens 64 are biconvex or plano-convex lenses. A cavity 67 extends between the input lens 62 and the output lens 64 and is void of any optical elements. Preferably, the relay group 61 has a magnification of -1 or 1 and is afocal.

The lenses 62, 64 may be identical with respect to their optical properties (i.e. material, size, radii, and/or structure), however, the lens 64 is rotated by 180 degrees with respect to the optical axis 9. The relay group 61 is symmetric (e.g. axial symmetric or mirror symmetric) around a symmetry axis 66 or symmetry plane, which is perpendicular to the optical axis 9 of the relay system 10. As shown in Fig. 11, the symmetry axis 66 or similarly, the symmetry plane, may be disposed at the center of the relay group 61.

Preferably, the lenses 62, 64 of the relay group 61 are selected and arranged such that the first and second intermediate images are generated from the first and second rays 23, 24 at an intermediate image plane 65 inside the relay group 61, in particular between the input lens 62 and the output lens 64. In some instances, the intermediate image plane substantially coincides with the symmetry axis 66 (not shown).

Notably, the relay groups 11, 41, 61 described above can be combined with each other within endoscope 1. For instance, the relay system 10 may comprise at least two relay groups 11, 41, 61 each having a different optical layout. In alternative embodiments, the relay system 10 comprises only relay groups 11, 41, 61 having the same optical layout.

The relay system 10 (including the relay groups 11 and/or relay groups 41 and/or the relay groups 61) may be configured such that the first rays 23 and the second rays 24 are coupled out of the relay system 10 at the proximal end portion 4. In particular, the first rays 23 and the second rays 24 may be spatially separated from each other at the proximal end portion 4. Furthermore, the first and second rays output from the first and second objectives 5, 6, wherein the marginal rays are parallel to the corresponding chief rays and coupled out of the relay system 10 have still marginal rays, which are parallel to the corresponding chief rays.

Table 1 below summarizes an exemplary set of lens data of the endoscope 1 having relay groups 11. It is noted that the prism assembly 29 of the second objective 6 is not incorporated in this table. However, a person skilled in the art is able to model the prism assembly 29 of the second objective 6.

As usual, the optical glasses used are designated by a numerical glass code. In Table 1, a ten-digit code is used. The first six digits correspond to the refractive index in terms of (n_{d}-1)x10⁶. The 4 decimals correspond to the Abbe number in terms of v_{d}x10². E.g. for n_{d}=1.5168, v_{d}=64.17 the glass code is 516800.6417.

**Table 1: lens data of the endoscope 1 having objective 5 and the relay system 10.**

| **Surface** | **Radius** | **Thickness** | **glass code** | **Non-Centered data** |
|---|---|---|---|---|
| Object | infinity | 110.000 | | |
| 1 | -5.176 | 0.301 | 640002.6021 | |
| 2 | 11.465 | 0.301 | 517602.6350 | |
| 3 | 13.743 | 9.085 | | |
| 4 | infinity | 0.301 | 753671.3750 | |
| 5 | 6.879 | 0.701 | 693495.5081 | |
| 6 | -9.502 | 3.000 | | |
| 7 | infinity | 0.000 | | Decenter Y=+/- 1.6 |
| 8 | 30.976 | 2.000 | 692109.5454 | |
| 9 | -13.225 | 2.000 | 749502.3495 | |
| 10 | -137.099 | 34.732 | | |
| 11 | 6.231 | 1.500 | 516800.6417 | |
| 12 | 10.689 | 35.305 | | |
| 13 | -133.873 | 0.750 | 740769.2779 | |
| 14 | 35.448 | 1.300 | 716998.4796 | |
| 15 | -36.146 | 0.439 | | |
| 16 | 43.892 | 1.300 | 585990.6105 | |
| 17 | -199.945 | 3.000 | | |
| 18 | 30.976 | 2.000 | 692109.5454 | |
| 19 | -13.225 | 2.000 | 749502.3495 | |
| 20 | -137.099 | 34.732 | | |
| 21 | 6.231 | 1.500 | 516800.6417 | |
| 22 | 10.689 | 35.305 | | |
| 23 | -133.873 | 0.750 | 740769.2779 | |
| 24 | 35.448 | 1.300 | 716998.4796 | |
| 25 | -36.146 | 0.439 | | |
| 26 | 43.892 | 1.300 | 585990.6105 | |
| 27 | -199.945 | 3.000 | | |
| 28 | 30.976 | 2.000 | 692109.5454 | |
| 29 | -13.225 | 2.000 | 749502.3495 | |
| 30 | -137.099 | 34.732 | | |
| 31 | 6.231 | 1.500 | 516800.6417 | |
| 32 | 10.689 | 35.305 | | |
| 33 | -133.873 | 0.750 | 740769.2779 | |
| 34 | 35.448 | 1.300 | 716998.4796 | |
| 35 | -36.146 | 0.439 | | |
| 36 | 43.892 | 1.300 | 585990.6105 | |
| 37 | -199.945 | 3.000 | | |
| 38 | 30.976 | 2.000 | 692109.5454 | |
| 39 | -13.225 | 2.000 | 749502.3495 | |
| 40 | -137.099 | 34.732 | | |
| 41 | 6.231 | 1.500 | 516800.6417 | |
| 42 | 10.689 | 5.860 | | |
| 43 | -19.079 | 0.995 | 755199.2751 | |
| 44 | 5.221 | 2.830 | 639999.6008 | |
| 45 | -7.624 | 0.075 | | |
| 46 | 4.719 | 2.000 | 750840.2769 | |
| 47 | 22.341 | 0.000 | | |

Exemplary lens data of the rod lenses 62, 64 of the relay group 61 are provided in table 2.

**Table 2: lens data of relay group 61.**

| **Surface** | **Radius** | **Thickness** | **glass code** |
|---|---|---|---|
| 1 | 15.91 | 34.95 | 585990.6105 |
| 2 | -15.1 | 8.41 | |
| 4 | 15.1 | 34.95 | 585990.6105 |
| 5 | -15.91 | 3.65 | |

The exemplary lens data of table 2 can be combined, optionally in slightly modified form, with the exemplary lens data of table 1, for instance, in case the endoscope 1 comprises at least one relay group 61 in addition to or in lieu of a relay group 11. It is also possible to combine the lens data of table 1, optionally in slightly modified form, with the exemplary lens data of table 2. By comparing the content of tables 1 and 2, as well as the associated Figures, the skilled person is capable of manufacturing the endoscope as defined by the appended set of claims.

The endoscope 1' differs from the endoscope 1 in that a tube lens group 30 is disposed after the first and second objectives 5, 6 instead of the relay system 10.

The endoscope 1' comprises an image sensor 31 at the distal portion of the shaft 2 and on the image side of the objectives 5, 6. The tube lens group 30 is arranged between the objectives 5, 6 and the image sensor 31. The tube lens group 30 is configured to project a first final image and/or a second final image onto the image sensor 31. To this end, the image sensor 31 may be disposed in the focal plane of the tube lens group 30. The tube lens group 30 has a positive focal length and may comprise solely one lens or a plurality of lenses.

A shutter 32 may be disposed between the objectives 5, 6 and the tube lens group 30. The shutter 32 can be used to switch between images from the first and second objectives 5, 6, thus allowing different directions of view 7, 8. If the first and second objectives 5, 6 have the same direction of view (not shown), the shutter 32 may switch between left and right creating a 3D image (stereoscopic image) using just one image sensor 31. By partially blocking the pupil plane of the objectives a user can switch between the images of the two objectives.

It is noted that various embodiments and features described above can be combined with each other as long as they do not contradict each other.

### Reference numerals

- 1: endoscope
- 1': endoscope
- 2: shaft
- 3: distal end portion
- 4: proximal end portion
- 5: first objective
- 6: second objective
- 7: first direction of view
- 8: second direction of view
- 9: optical axis
- 10: relay system
- 11: relay group
- 12: input lens group
- 13: field lens
- 14: output lens group
- 15: intermediate image plane
- 16: pupil plane
- 17: cavity
- 18: shutter
- 19: eye piece
- 20: infinity space
- 21: first optical axis
- 22: second optical axis
- 23: first rays
- 24: second rays
- 25: first focal plane
- 26: second focal plane
- 27: input lens group
- 28: output lens group
- 29: prism assembly
- 30: tube lens group
- 31: image sensor
- 32: shutter
- 33: front tip
- 41: relay group
- 42: input lens group
- 44: output lens group
- 45: symmetry axis
- 46: field lens
- 48: field lens
- 51: endoscope
- 52: objective
- 53: relay system
- 54: eye piece
- 55: intermediate images
- 56: object
- 61: relay group
- 62: input lens
- 64: output lens
- 65: intermediate image plane
- 66: symmetry axis
- 67: cavity
- d1: distance
- d2: distance
- p1: light rays
- p2: light rays
- p3: light rays
- p4: light rays
- p5: light rays
- p6: light rays

## Claims

1. Endoscope (1, 1'), comprising:
a shaft (2) having a distal end portion (3) and a proximal end portion (4),
a first objective (5) arranged at the distal end portion (3) and having a first direction of view (7), and a first optical axis (21), the first objective (5) being set up to generate an object image in infinity,
a second objective (6) arranged at the distal end portion (3) and having a second direction of view (8), and a second optical axis (22), the second objective (6) being set up to generate an object image in infinity, and
a lens system (10, 30) disposed within the shaft (2) between the distal end portion (3) and the proximal end portion (4), the lens system (10, 30) having an optical axis (9),
wherein the first objective (5) and the second objective (6) are both optically coupled to the same lens system (10, 30) such that first parallel rays from the first objective (5) and second parallel rays from the second objective (6) are input into the lens system (10, 30),
wherein the lens system (10) is an optical relay system (10) comprising at least one relay group (61), wherein the at least one relay group (61) consists of a single input lens (62) and a single output lens (64), and a cavity (67) void of optical elements extends between the input lens (62) and the output lens (64).

2. Endoscope (1, 1') according to claim 1, wherein a distance (d1) between the first objective (5) and the lens system (10, 30) differs from a distance (d2) between the second objective (6) and the lens system (10, 30).

3. Endoscope (1, 1') according to any one of the preceding claims, wherein the first direction of view (7) differs from the second direction of view (8), wherein the first optical axis (21) and the second optical axis (22) are parallel to each other.

4. Endoscope (1,1') according to any one of the preceding claims, wherein all optical components of the first (5) and second objectives (6) are fixed relatively to the shaft (2).

5. Endoscope (1, 1') according to any one of the preceding claims, wherein the first direction of view (7), the second direction of view (8) and the optical axis (9) of the lens system (10, 30) are tilted relative to each other and do not lie in the same plane.

6. Endoscope (1, 1') according to any one of the preceding claims, configured such that the chief rays coming from different object points and output from the objectives (5,6) are not parallel to each other and/or are not parallel to the optical axis (9) of the lens system (10, 30).

7. Endoscope (1,1') according to any one of the preceding claims, wherein the first optical axis (21) is parallel to the first direction of view (7) and/or wherein the second optical axis (22) is not parallel to the second direction of view (8).

8. Endoscope (1) according to any one of the preceding claims, wherein the magnification of the relay system (10) is -1 or 1 and/or the magnification of the at least one relay group (11, 41,61) is -1 or 1 and/or, wherein the relay system (10) is afocal and/or the at least one relay group (11, 41, 61) is afocal.

9. Endoscope (1) according to any one of the preceding claims, wherein the relay system (10) is configured to generate first and second intermediate images (15) from the first and second rays, respectively, wherein the first and second intermediate images (15) are generated within the at least one relay group (11, 41, 61) and at least partially overlay, preferably concentrically overlay.

10. Endoscope (1) according to any one of the preceding claims, configured such that the first and second rays are coupled out of the relay system (10) at the proximal end portion (4), wherein the first and second rays are spatially separated from each other at the proximal end portion (4).

11. Endoscope (1) according to any one of the preceding claims, including at least one further relay group (11), wherein the at least one further relay group (11) comprises an input lens group (12), an output lens group (14), a cavity defined between the input lens group (12) and the output lens group (14), and a field lens (13) disposed in the cavity.

12. Endoscope (1) according to any one of the preceding claims, wherein the input lens (62) is a rod lens and/or the output lens (64) is a rod lens, wherein the input lens (62) and the output lens (64) are preferably made of the same material and/or biconvex or plano-convex.

13. Endoscope (1) according to claim 12, wherein first and second intermediate images (65) are generated within the relay group (61) between the input lens (62) and the output lens (64) such that the first and second intermediate images have a size, which is smaller than a maximum diameter of all lenses (62, 64) of the relay group (61).

14. Endoscope (1) according to any one of the preceding claims, wherein the relay system (10) comprises a plurality of relay groups (11, 41, 61) that are arranged in series, wherein each relay group (11) is configured to generate first and second intermediate images (15) within the respective relay group (11, 41, 61), wherein all intermediate images generated in the relay system (10) have the same paraxial size.

15. Endoscope (1) according to any one of the preceding claims, wherein the at least one relay group (41, 61) is axial symmetric around a symmetry axis (45, 66), which is perpendicular to the optical axis (9) of the relay system (10), wherein the symmetry axis (45, 66) is disposed in the center of the relay group (41, 61).
